Europäisches Patentamt

European Patent Office

Office européen des brevets

(11) Publication number: **0 280 528**
**A2**

## EUROPEAN PATENT APPLICATION

(21) Application number: **88301581.0**

(22) Date of filing: **24.02.88**

(51) Int. Cl.4: **A 61 M 25/00**

(30) Priority: **26.02.87 GB 8704531**

(43) Date of publication of application:
**31.08.88 Bulletin 88/35**

(84) Designated Contracting States:
**AT BE CH DE ES FR GB GR IT LI LU NL SE**

(71) Applicant: **BARD LIMITED**
**Pennywell Industrial Estate**
**Sunderland SR4 9EW (GB)**

(72) Inventor: **Brazier, Gary Brian**
**10 Main Road Great Oakley**
**Harwich Essex CO12 5AZ (GB)**

(74) Representative: **Meeks, Frank Burton et al**
**BREWER & SON 5-9 Quality Court Chancery Lane**
**London WC2A 1HT (GB)**

(54) Catheter introducer.

(57) Apparatus for introducing a catheter into a bodily cavity includes a plastics sheath (20) having an internal bore sufficiently large to accommodate a catheter tube (31). Two parallel lines of weakness (24 and 25) extend along the length of the sheath (20) to define a tear-off strip (26). A hand-grippable tab (27) is located at the near end (21) of the strip (26), and a tab (28) is located at the near end of the sheath (20) opposite the tab (27).

The sheath (20) is carried by a trocar (10) which makes a snug sliding fit within the sheath (20). The trocar (10) carrying the sheath (20) is stabbed through the abdominal wall of the patient until the needle point 11 enters the bladder cavity. The needle is then removed, and the distal end (30) of a catheter is passed through the sheath bore (29) into the bladder cavity. The sheath (20) is pulled from the patient's body using the tab (28), and the sheath (20) is then stripped from the catheter tube (31) by pulling apart the tabs (27 and 28) to remove the strip (26) from the rest of the sheath (20).

FIG. 1.

EP 0 280 528 A2

**Description**

CATHETER INTRODUCER

This invention relates to a method of, and apparatus for, introducing a catheter into a bodily cavity, more particularly but not exclusively a suprapubic catheter into a bladder cavity.

The abdominal wall is tough, and will tend to close any hole in it made for insertion of a suprapubic catheter. If a sheath is used for holding open the hole for introduction of a suprapubic catheter, there is then a problem how to remove the sheath after the catheter has been introduced. The present invention seeks to provide a solution to this problem which makes the introduction of suprapubic catheters more efficient and cost effective than with previous proposals.

According to the present invention there is provided apparatus for introducing a catheter into a bodily cavity, the apparatus comprising a sheath with an internal bore large enough to accommodate a catheter tube, and a trocar which is a snug sliding fit within the sheath and which serves to carry the sheath into a catheter introduction position in which an inner end of the sheath is in the bodily cavity and an outer end of the sheath is external to the body, the trocar being removable from the sheath when in the catheter introduction position to allow advancement of the catheter tube down the bore of the sheath and into the bodily cavity; wherein the sheath is formed with two parallel lines of weakness along its length, to define between the lines a tear-off strip, said strip having a hand-grippable element for pulling on the strip and thereby parting it from the rest of the sheath at the two lines of weakness so that, after introduction of the catheter, the sheath may be withdrawn from the body of the patient, and thereafter the hand-grippable element used to part the tear-off strip from the rest of the sheath so allowing the sheath to be removed from around the catheter tube.

Preferably, the sheath is made as an injection moulding from a plastics material and has the form of a thin-walled tube, with two parallel lines of weakness running down one side of the tube, and with two hand-grippable elements at one end of the tube, one on each side of the tube, one on the tear-off strip between the two lines of weakness, and one on the larger portion of the tube.

Polypropylene, polyurethane and polyethylene are examples of three suitable materials for the sheath, and polyethylene is the preferred material.

It is contemplated that the sheath will be of a diameter appropriate to accommodate any cylindrical suprapubic catheter for example a Foley catheter or a pigtail catheter. Typically the length will be of the order of 15 to 20 cms.

Preferably, the shaft of the trocar has a longitudinally extending liquid flow channel, in order that penetration of the point of the trocar into the bladder is signalled externally of the body of the patient by "flash back" of urine along the channel to the near end of the sheath. It may be convenient to provide with the sheath a small plug for plugging the near end of the sheath after the trocar has been withdrawn from the sheath and before the catheter tube is introduced down the bore of the sheath.

For a better understanding of the invention, and to show more clearly how the same may be carried into effect, reference will now be made, by way of example, to the accompanying drawings, in which:

Figure 1 is a view, partly in section, of a trocar and sheath in accordance with the invention; and

Figure 2 is a view from the side of a Foley catheter with its tube extending through the bore of the sheath.

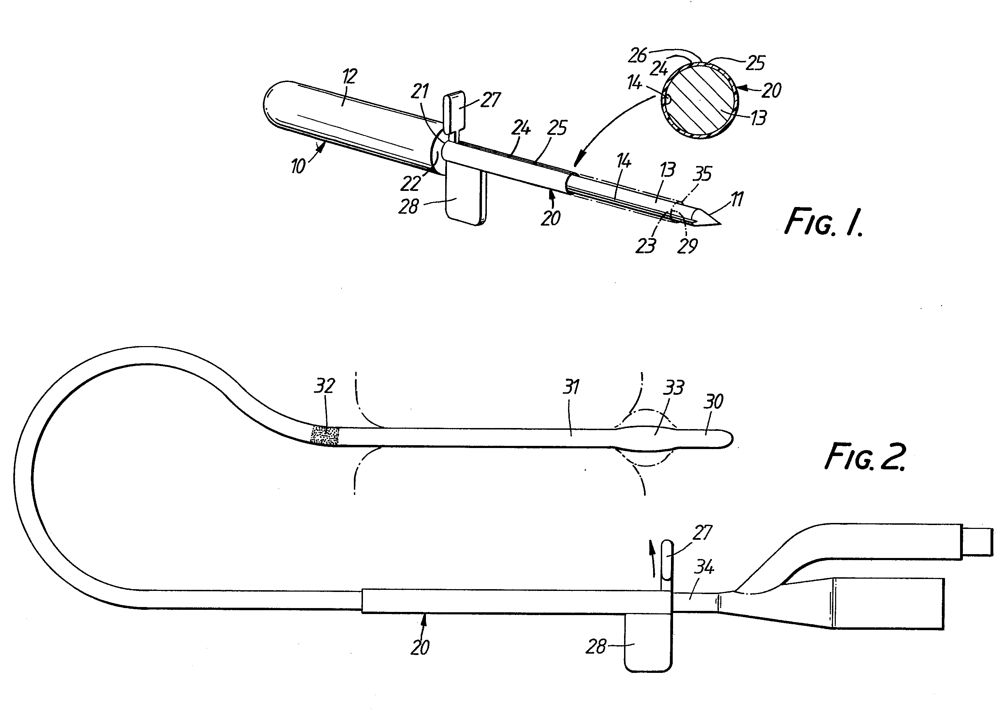

Referring to Figure 1, the trocar 10 has a sharp point 11 and a handle 12. Along the surface of its shaft 13 runs a narrow liquid flow channel 14. The channel 14 can alternatively be provided as a flat on the surface of the shaft 13, or indeed in any other way which will signal penetration of the point 11 into the bladder by a "flash-back" of urine to the handle end of the trocar 10.

The sheath 20 is made of polyethylene and fits snugly over the shaft 13 of the trocar so that a near end 21 of the sheath abuts the shaft end 22 of the handle 12 of the trocar, and the other end 23 of the sheath lies closely adjacent to the point 11 of the trocar. Running along the length of the sheath 20 are two lines of weakness 24 and 25 where the wall thickness is substantially reduced. A tear-off strip 26 lying between the two lines of weakness has, at its near end 21, a hand-grippable tab 27. On the opposite side of the sheath from the tab 27 there is another tab 28. The general thickness of the wall of the sheath 20 is as small as possible, but it has to be large enough to resist pressure from the tough abdominal wall of the patient which would otherwise crush the sheath 20. Preferably, the end 23 of sheath is chamfered as at 35.

In use of the apparatus, an incision in the abdominal wall is made by a suitable cutting instrument, and then the assembly of trocar and sheath 20 is stabbed through the wall until the point 11 of the trocar enters the bladder cavity, giving rise to a flash-back of urine along the tube 14.

At this point, the trocar 10 is removed, to leave the sheath 20 in position, connecting the bladder cavity with the exterior of the body. If desired, a small plug can be placed in the near end 21 of the open bore 29 of the sheath to prevent further outward flow of urine.

Referring now to Figure 2, the next stage is to withdraw the plug and insert down the bore 29 of the sheath 20 the distal end 30 of a Foley or other catheter, and so much of the length of the catheter tube 31 of the catheter as will bring the end 30 into the bladder cavity. A mark 32 on the wall of the catheter tube 31 can be relied upon, if desired, to indicate correct placement. With the catheter correctly positioned, in the case of a Foley catheter its bulb 33 is inflated in order to retain the distal end 30 in the bladder cavity, and then the sheath 20 is pulled, using the tab 28, from the body of the patient, and along the catheter tube 31 to the near end 34 of the tube. The abdominal wall closes around the exterior surface of the catheter tube 31. If the catheter tube is supported from within by a stylet, then this is removed at this stage.

The sheath 20 is stripped from the catheter tube 31 by gripping the tab 28 in one hand and the tab 27 in the other hand, and pulling the two tabs away from each other. This causes the tear-off strip 26 to part from the rest of the sheath 20 by tearing along the lines of weakness 24 and 25. Once the tear-off strip is separated from the rest of the sheath 20, the sheath can easily be removed from the catheter tube 31.

## Claims

1. Apparatus for introducing a catheter into a bodily cavity, the apparatus comprising a sheath (20) with an internal bore large enough to accommodate a catheter tube (31), and a trocar (10) which is a snug sliding fit within the sheath (20) and which serves to carry the sheath (20) into a catheter introduction position in which an inner end of the sheath (20) is in the bodily cavity and an outer end of the sheath is external to the body, the trocar (10) being removable from the sheath (20) when in the catheter introduction position to allow advancement of the catheter tube (31) down the bore of the sheath (20) and into the bodily cavity; characterised in that the sheath (20) is formed with two parallel lines of weakness (24 and 25) along its length, to define between the lines a tear-off strip (26), said strip (26) having a hand-grippable element (27) for pulling on the strip (26) and thereby parting it from the rest of the sheath (20) at the two lines of weakness (24 and 25) so that, after introduction of the catheter, the sheath (20) may be withdrawn from the body of the patient, and thereafter the hand-grippable element (27) used to part the tear-off strip (26) from the rest of the sheath (20) so allowing the sheath (20) to be removed from around the catheter tube (31).

2. Apparatus as claimed in claim 1 wherein the sheath (20) is made of a plastics material.

3. Apparatus as claimed in claim 2 wherein the sheath (20) is made as an injection moulding.

4. Apparatus as claimed in any preceding claim wherein the sheath (20) has the form of a thin-walled tube, with the two parallel lines of weakness (24 and 25) running down one side of the tube.

5. Apparatus as claimed in claim 4 wherein the hand-grippable element (27) and a further hand-grippable element (28) are located at one end of the tube on opposite sides of the tube.

6. Apparatus as claimed in any preceding claim wherein the trocar (10) has a liquid flow channel extending longitudinally thereon.

7. Apparatus as claimed in any one of claims 1 to 5 wherein the trocar (10) has a flat on its surface extending longitudinally thereon.

8. Apparatus as claimed in any preceding claim further comprising a plug to plug the near end of the sheath (20).